(19) Europäisches Patentamt

European Patent Office

Office européen des brevets

(11) **EP 2 944 725 A1**

(12) **DEMANDE DE BREVET EUROPEEN**

(43) Date de publication:
**18.11.2015 Bulletin 2015/47**

(21) Numéro de dépôt: **15167264.9**

(22) Date de dépôt: **12.05.2015**

(51) Int Cl.:
*E02D 1/02* *(2006.01)*     *G01N 3/30* *(2006.01)*
*G01N 3/34* *(2006.01)*     *E21B 49/00* *(2006.01)*
*G01N 11/12* *(2006.01)*     *G01N 33/24* *(2006.01)*

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA**

(30) Priorité: **13.05.2014 FR 1454251**

(71) Demandeur: **Sol Solution**
**63200 Riom (FR)**

(72) Inventeurs:
• **Benz Navarrete, Miguel**
**63000 CLERMONT-FERRAND (FR)**
• **Gourves, Roland**
**63200 MARSAT (FR)**

(74) Mandataire: **Grand, Guillaume et al**
**Cabinet Lavoix**
**62, Rue de Bonnel**
**69003 Lyon (FR)**

(54) **PÉNÉTROMÈTRE DYNAMIQUE, ENSEMBLE DE MESURE, SYSTÈME ET MÉTHODE DE DÉTERMINATION DE LA COMPACITÉ ET DE LA CAPACITÉ PORTANTE D'UN SOL**

(57) Ce pénétromètre dynamique (1) comprend une tête de battage (3), qui est prolongée par une barre (4) terminée par une pointe (5) prévue pour pénétrer dans un sol (S), et des moyens de mesure pour déterminer à la fois l'énergie de battage, appliquée sur la tête de battage, et l'enfoncement de la pointe dans le sol. Les moyens de mesure sont portés en intégralité par la tête de battage et sont adaptés pour mesurer l'accélération et/ou la vitesse de la tête de battage.

FIG.1

**Description**

La présente invention concerne un pénétromètre dynamique, un ensemble de mesure de la compacité et de la capacité portante d'un sol comprenant un tel pénétromètre, ainsi qu'une méthode de détermination de la compacité et de la capacité portante d'un sol associée.

Il est connu d'utiliser un pénétromètre dynamique pour la reconnaissance des sols en surface, et plus précisément pour le contrôle de la compacité ou de la portance des couches de sol de différents ouvrages en terre.

Un essai de pénétration dynamique consiste à faire pénétrer dans le sol, par battage, un train de tiges ou de tubes métalliques muni d'une pointe conique.

Un pénétromètre dynamique léger à énergie constante est un appareil de construction relativement simple, composé d'une barre d'essai dont l'extrémité inférieure est munie d'une pointe conique dont l'angle au sommet varie à 30 à 90°, qui est prévue pour pénétrer dans le sol. A l'extrémité supérieure de la barre d'essai, le pénétromètre dynamique léger est muni d'une enclume prévue pour recevoir les chocs répétés d'une masse de battage qui coulisse le long d'une barre de guidage.

Le battage, c'est-à-dire l'enfoncement de la pointe conique dans le sol, est réalisé en levant puis en lâchant la masse de battage de manière répétée. La masse de battage tombe d'une hauteur fixe, et fournit donc au pénétromètre une énergie constante. La masse de battage des pénétromètres dynamiques légers est généralement inférieure ou égale à 10 kg. La hauteur de chute moyenne est de l'ordre de 55 cm. L'énergie de battage est donc de l'ordre de 50 J.

Pour exploiter les résultats d'un tel essai, on enregistre généralement le nombre de coups nécessaires pour enfoncer la pointe conique d'une profondeur donnée. Cette profondeur est mesurée par l'opérateur grâce à des repères de mesure présents sur la barre d'essai ou simplement à l'aide d'un mètre ou d'une règle.

L'utilisation d'un tel pénétromètre est relativement fastidieuse, car il est nécessaire de compter le nombre de coups nécessaires pour enfoncer la pointe d'une hauteur donnée, et de noter ce résultat sur une feuille dite « de chantier ». De plus, la résolution verticale des mesures est très limitée car le nombre de coups est relevé uniquement tous les 10 ou 20 cm d'enfoncement.

US-A-2007/0277598 divulgue un pénétromètre dynamique léger à énergie constante dans lequel la mesure de l'enfoncement de la pointe conique est automatisée grâce à un faisceau laser mesurant la profondeur de pénétration dans le sol. Ceci permet de faciliter l'essai de pénétration dynamique, en automatisant la mesure de l'enfoncement. Toutefois, le temps de mise en place d'un essai à l'aide d'un tel pénétromètre est relativement élevé, et l'essai est limité à une énergie de battage constante, puisqu'il n'est pas prévu de mesurer la vitesse ou la hauteur de chute de la masse. De plus, la fiabilité de l'essai est limitée puisqu'il est possible de laisser tomber la masse de battage d'une hauteur de chute inférieure à la hauteur prévue.

Par ailleurs, l'utilisation d'une énergie de battage constante pose problème dans le cas des sols mous, car l'énergie de battage est relativement importante et l'enfoncement à chaque coup est relativement élevé. Par conséquent, la précision de la mesure et l'interprétation des valeurs obtenues sont dégradées. Dans le cas des sols raides, l'énergie de battage est insuffisante pour enfoncer la pointe dans le sol, et par conséquent l'essai ne peut pas être effectué.

Les pénétromètres dynamiques à énergie variable sont une alternative aux pénétromètres dynamiques à énergie constante. L'énergie de battage est produite par un opérateur qui frappe le pénétromètre à l'aide d'un maillet, et la force de frappe varie en fonction de la dureté de sols. Des jauges de contrainte ou d'autres moyens de mesure similaires, installés dans l'enclume du pénétromètre, permettent de mesurer l'énergie de battage fournie pour chaque frappe. Un boîtier électronique muni d'un codeur enregistre l'enfoncement pour chaque frappe, grâce à des moyens de mesure disposés au sol. Un exemple d'un tel pénétromètre est divulgué dans FR-A-2 817 344.

Un autre exemple d'un pénétromètre dynamique à énergie variable est donné par WO-A-2013/124426 qui peut être considéré comme l'état de la technique le plus proche de l'invention définie aux revendications 1 et 13. WO-A-2013/124426 divulgue un pénétromètre comportant une tête de battage, prolongée par une barre terminée par une pointe. Aux fins de la détermination de l'énergie de battage, appliquée sur la tête de battage, ce pénétromètre comporte deux capteurs de déformation, notamment deux jauges de contrainte, qui sont portées par la tête de battage : comme rappelé dans la partie introductive de WO-A-2013/124426, l'utilisation de ces jauges de contrainte permet de déduire l'énergie de battage. Aux fins de la détermination de l'enfoncement de sa pointe dans le sol, le pénétromètre de WO-A-2013/124426 comprend une courroie, qui s'étend jusqu'à un boîtier, posé au sol et servant de guide à la pointe, cette courroie n'étant donc pas intégralement portée par la tête de battage : comme expliqué dans WO-A-2013/124426, la courroie permet de mesurer directement la distance parcourue par la pointe lors de son enfoncement. On notera par ailleurs que WO-A-2013/124426 envisage, à titre uniquement optionnel, d'intégrer à sa tête de battage un accéléromètre : cette accéléromètre sert de dispositif de mesure de secours en cas de défaillance d'une des deux jauges de contrainte, ou bien réalise une mesure redondante de l'accélération, cette dernière étant normalement calculée à l'aide, à la fois, des mesures de déformation, réalisées par les deux jauges de contrainte, et de la mesure de la distance d'enfoncement, réalisée par la courroie. Ainsi, l'enseignement technique de WO-A-2013/124426 prévoit nécessairement d'associer l'accéléromètre optionnel précité avec au moins une des deux jauges de contrainte et avec la courroie.

On comprend donc que les pénétromètres dynamiques à énergie variable nécessitent au moins deux moyens de mesure distincts, dont l'un mesure l'énergie de battage et l'autre la profondeur d'enfoncement, ce qui les rend complexes, coûteux et contraignants. Il en résulte que leur prix initial est élevé et que le temps pour les installer avant la réalisation d'un essai est important du fait qu'il est nécessaire, lors de la mise en station, de connecter chaque élément de mesure au boitier et au PC d'acquisition.

C'est à ces inconvénients qu'entend plus particulièrement remédier l'invention, en proposant un nouveau pénétromètre dynamique de construction simple, permettant d'améliorer la technique de pénétration pour des auscultations d'une profondeur inférieure à 2 m et ayant des applications notamment dans le domaine du génie civil, agricole ou militaire.

A cet effet, l'invention a pour objet un pénétromètre dynamique, tel que défini à la revendication 1.

Grâce à l'invention, le pénétromètre dynamique est équipé uniquement de moyens de mesure de l'accélération et/ou de la vitesse, par exemple un unique capteur d'accélération ou un unique géophone, ce qui permet d'automatiser et donc de fiabiliser l'essai de mesure de la compacité du sol. L'accélération ou la vitesse permettent à la fois de calculer l'énergie de battage et l'enfoncement de la pointe pour chaque coup. Aucun capteur supplémentaire n'est placé par terre, ce qui permet de réduire le temps d'installation nécessaire à la mise en place de l'essai.

Des aspects avantageux mais non obligatoires de l'invention sont spécifiés aux revendications 2 à 12.

L'invention concerne en outre une méthode de mesure de la compacité et de la capacité portante d'un sol, telle que définie à la revendication 13.

Des aspects avantageux en sont spécifiés aux revendications 14 et 15.

L'invention sera mieux comprise et d'autres avantages de celle-ci apparaîtront plus clairement à la lumière de la description qui va suivre d'un pénétromètre dynamique, d'un ensemble de mesure et d'une méthode associée conformes à l'invention, donnée uniquement à titre d'exemple et faite en référence aux dessins annexés dans lesquels :

- la figure 1 est une vue en perspective éclatée d'un ensemble de mesure de la compacité d'un sol, comprenant un pénétromètre dynamique et un dispositif de battage conformes à l'invention ;
- la figure 2 montre un maillet prévu pour être utilisé avec le pénétromètre, lorsque le dispositif de battage n'est pas utilisé ;
- la figure 3 est une vue à plus grande échelle d'une pointe faisant partie du pénétromètre de la figure 1 ;
- la figure 4 est une variante de la pointe de la figure 3 ;
- la figure 5 est une coupe d'une tête de mesure du pénétromètre de la figure 1 ;

- la figure 6 est une coupe d'une masse faisant partie du dispositif de battage de la figure 1 ;
- la figure 7 est une coupe d'une colonne de battage faisant partie du dispositif de battage de la figure 1 ;
- la figure 8 est une coupe de l'ensemble de la figure 1, en configuration assemblée ; et
- les figures 9 à 12 sont des graphiques montrant l'évolution de paramètres déterminés à partir de mesures réalisées au moyen du pénétromètre ou de l'ensemble de mesure, relatifs à la compacité du sol.

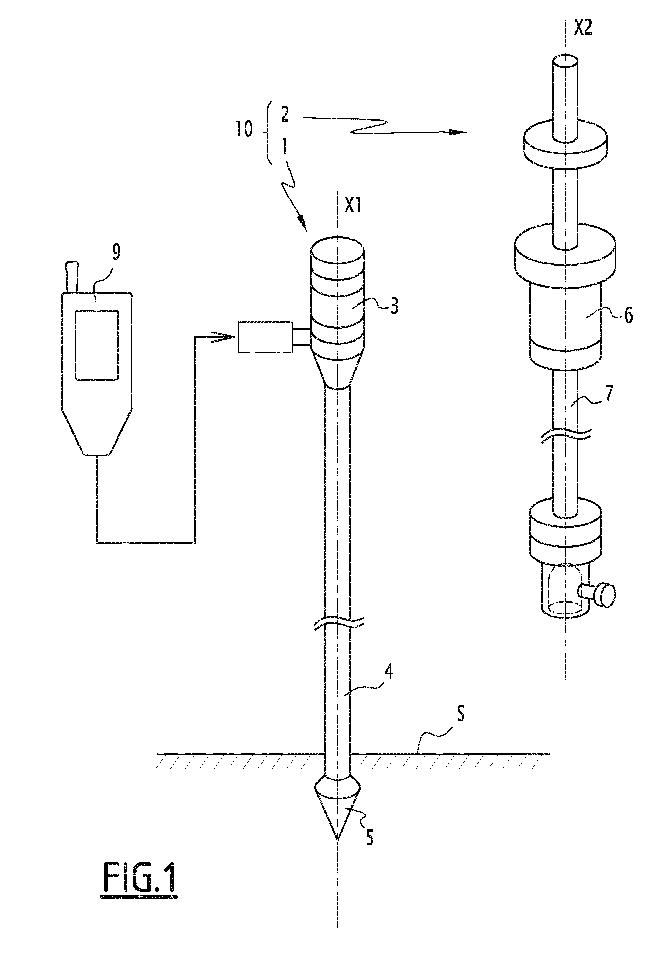

La figure 1 montre un ensemble 10 de mesure de la compacité d'un sol S, qui comprend un pénétromètre dynamique 1, ainsi qu'un dispositif de battage 2 conçu pour être assemblé, de manière optionnelle avec le pénétromètre 1.

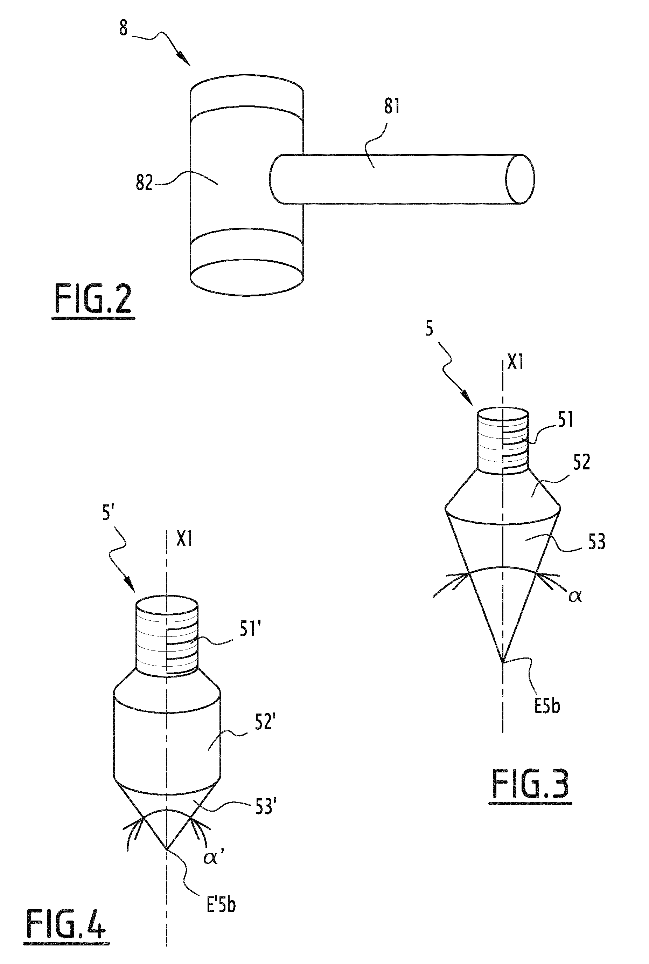

Lorsque le pénétromètre 1 est utilisé sans le dispositif de battage 2, il s'agit d'un pénétromètre à énergie variable et un maillet 8 représenté à la figure 2 est prévu pour impacter le pénétromètre 1.

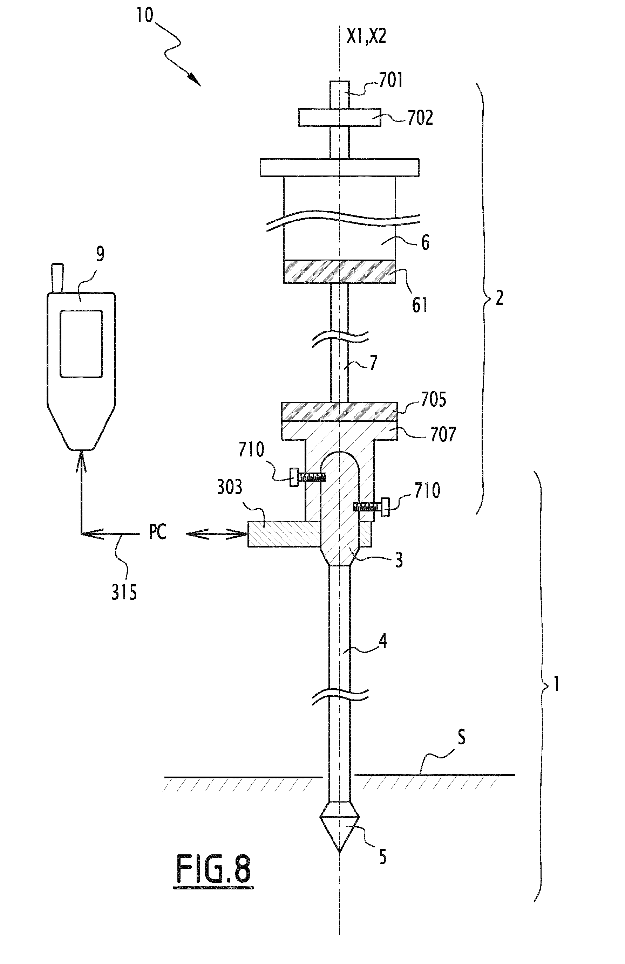

Lorsque le dispositif de battage 2 est assemblé au pénétromètre 1, l'ensemble 10 ainsi formé constitue un pénétromètre dynamique à énergie constante, représenté à la figure 8 en configuration assemblée. Le maillet 8 n'est alors pas utilisé.

Le pénétromètre 1 s'étend selon un premier axe longitudinal X1, positionné verticalement lorsque le pénétromètre 1 est utilisé pour réaliser un essai de pénétration dans un sol horizontal S.

Dans la suite, les termes « inférieur », « supérieur », « bas », « haut », « dessous » et « dessus » sont définis par rapport à cette orientation d'utilisation standard du pénétromètre 1, qui correspond à l'orientation des figures 1 et 3 à 8.

Le pénétromètre 1 comprend une tête de battage 3 dont l'extrémité inférieure E3b est prolongée par une barre 4. Une extrémité inférieure E4b de la barre 4 est prolongée par une pointe conique 5 destinée à pénétrer dans le sol S.

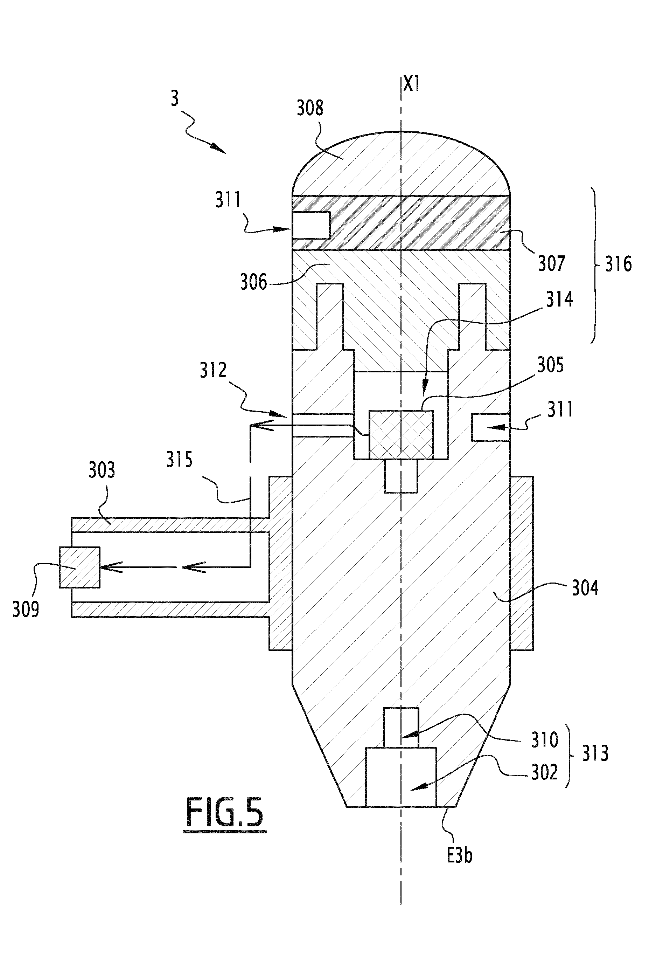

La figure 5 montre plus en détail la tête de battage 3, qui comprend un corps 304 de forme globalement cylindrique et à section circulaire, centré sur l'axe X1.

Des moyens de préhension, à savoir une poignée 303, sont assemblés au corps 304 et permettent à un opérateur de maintenir le pénétromètre 1, ou l'ensemble 10 lorsque le dispositif de battage 2 est utilisé, dans la position souhaitée pour réaliser l'essai.

L'extrémité inférieure E3b de la tête de battage 3 comporte un orifice 313 qui comprend une première partie 302, formant des moyens d'assemblage de la tête de battage 3 avec la barre 4, dans laquelle est logée une extrémité supérieure E4a de la barre 4. La première partie 302 de l'orifice 313 débouche sur l'extrémité inférieure E3b de la tête de battage 3, et est prolongée vers le haut par une deuxième partie 310 de l'orifice 313, de diamètre inférieur, qui forme des moyens de fixation pour la barre 4. La fixation de la barre 4 avec la tête de battage 3 est réalisée par vissage, par clipsage ou par goupillage.

Le corps 304 de la tête de battage 3 comporte une cavité 314 qui débouche vers le haut, centrée sur l'axe X1. Des moyens de mesure de l'accélération et/ou de la vitesse, par exemple un unique accéléromètre 305 et/ou un unique géophone, sont situés à l'intérieur de la cavité 314. Il peut s'agir par exemple d'un accéléromètre miniature, tel qu'un accéléromètre piézorésistif ou piézoélectrique. L'accéléromètre 305 mesure l'accélération du pénétromètre 1 selon l'axe X1. L'accéléromètre 305 est fixé à la tête de mesure, par exemple, par collage ou par vissage. Le capteur de mesure de vitesse mesure la vitesse du pénétromètre 1 selon l'axe X1.

La suite de la description concerne le mode de réalisation dans lequel un unique accéléromètre est utilisé.

La cavité 314 comporte un orifice de sortie 312 orienté radialement, débouchant sur l'extérieur de la tête 3, au travers duquel s'étend un câble électrique 315 relié à un élément de connexion 309 fixé par exemple au niveau de l'extrémité libre de la poignée 303. L'élément de connexion 309 permet la connexion électrique du pénétromètre 1 à des moyens de traitement d'un signal fourni par l'accéléromètre 305, par exemple un poste d'analyse 9, tel qu'un téléphone intelligent, un ordinateur personnel ou une tablette. La connexion entre l'élément de connexion 309 et le poste d'analyse 9 est filaire, et s'effectue par exemple au moyen d'un câble USB, ou sans fil, par exemple par ondes WIFI ou Bluetooth.

L'ouverture supérieure de la cavité 314 de la tête de battage 3 est refermée par une partie inférieure 306 d'une enclume 316. Un premier élément d'amortissement 307 est intercalé, le long de l'axe X1, entre la première partie 306 de l'enclume 316 et une deuxième partie 308 de l'enclume 316, également appelée « casque ». Les parties 306 et 308 sont réalisées par exemple à partir d'un alliage métallique tel qu'un alliage d'acier ou d'aluminium.

Le premier élément d'amortissement 307 est réalisé par exemple à partir de caoutchouc, d'une matière plastique, de polyuréthane ou des ressorts. L'élément d'amortissement 307 permet d'amortir les vibrations transmises lors de l'impact, ce qui augmente la durée du choc et diminue les hautes fréquences des vibrations entraînant le bruit dans un choc de type acier-acier.

La tête 3 est équipée de moyens de fixation du dispositif de battage 2 sur le pénétromètre 1, par exemple des orifices 311 orientés radialement et décalés le long X1. Un premier orifice 311 est ménagé dans le corps 304, et un deuxième orifice est ménagé dans l'élément d'amortissement 307.

La figure 2 montre le maillet 8, qui est prévu pour impacter le casque 308 du pénétromètre 1. Le maillet 8 comprend un manche 81 prolongé par une tête massive 82, réalisée par exemple à partir d'un alliage métallique et comportant dans ses extrémités des embouts en plastique, caoutchouc ou polyuréthane

La figure 3 montre plus en détail la pointe conique 5 du pénétromètre. La pointe 5 comprend une partie de fixation 51 prévue pour être raccordée à un porte-pointe situé au niveau de l'extrémité inférieure E4b de la barre 4. La partie de fixation 51 est prolongée vers le bas par une partie intermédiaire 52 tronconique, qui s'évase vers le bas, en s'éloignant de la partie de fixation 51. Une partie conique 53 prolonge la partie intermédiaire 52 vers le bas, à l'apposé de la partie de fixation 51, et comporte une extrémité en pointe E5b qui pénètre dans le sol S en premier lors d'un essai.

On note α, l'angle au sommet de la partie conique 53. De préférence, l'angle α est compris entre 30° et 90°.

Le pénétromètre 1 est un dispositif monobloc lors d'un essai, c'est-à-dire que la tête 3, la barre 4 et la pointe 5 sont fixés entre eux par défaut, de sorte que lors d'un essai, aucune opération d'assemblage préalable n'est nécessaire. En dehors des essais, il est possible de désassembler la tête 3, la barre 4 et la pointe 5.

La figure 4 montre une pointe 5' prévue pour être assemblée à la barre 4 à la place de la pointe 5. La partie intermédiaire 52' de la pointe 5' est de forme cylindrique à section circulaire et est prolongée vers le haut par une partie de fixation 51' analogue à la partie de fixation 51 de la pointe 5. Une partie conique 53' prolonge la partie intermédiaire 52' vers le bas, et comporte une extrémité en pointe E'5b. On note α' l'angle au sommet de la partie conique 53'. De préférence, l'angle α' est compris entre 30° et 90°

Le dispositif de battage 2 comprend une masse 6 mobile en translation, prévue pour coulisser le long d'une colonne de battage 7 s'étendant le long d'un deuxième axe longitudinal X2.

Lorsque le dispositif de battage 2 est assemblé au pénétromètre 1, les axes X1 et X2 sont alignés et on obtient un ensemble 10 de mesure de la compacité du sol S, représenté à la figure 8, équivalent à un pénétromètre dynamique à énergie constante. Le maillet 8 est alors remplacé par la masse 6 pour réaliser les impacts.

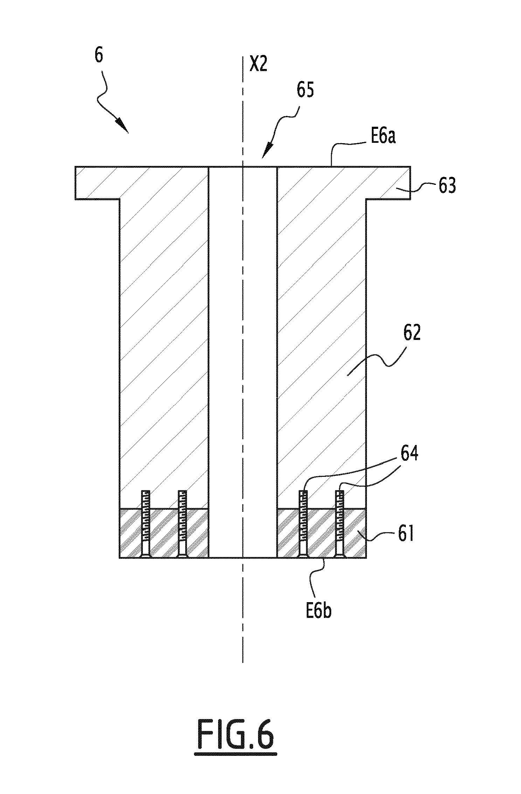

La masse 6, représentée à la figure 6, est de forme globalement cylindrique à section circulaire, et est centrée sur l'axe X2.

On note E6b, une extrémité inférieure de la masse 6, formée par un deuxième élément d'amortissement 61 réalisé par exemple à partir de caoutchouc ou d'une matière plastique. Un corps de masse 62, réalisé par exemple à partir d'un alliage métallique tel qu'un acier ou un alliage d'aluminium, est fixé au deuxième élément d'amortissement 61 au moyen d'éléments de fixation 64, tels que des vis. La masse 6 est traversée de part en part par un alésage central 65 centré sur l'axe X2.

On note E6a, une extrémité supérieure de la masse 6, formée par des seconds moyens de préhension 63 constitués par une collerette périphérique qui fait saillie radialement vers l'extérieur.

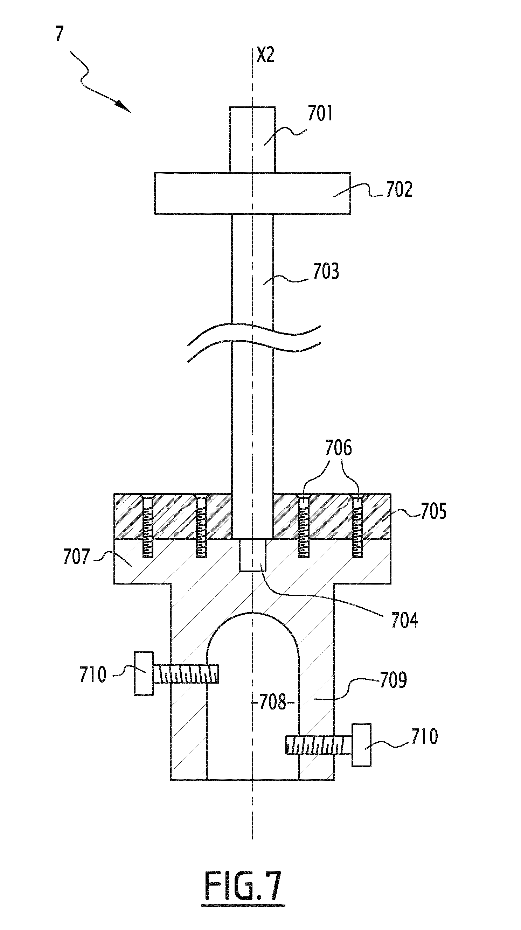

La figure 7 représente le dispositif de battage 2 non équipé de la masse 6. Une barre de guidage 703, centrée sur l'axe X2, comporte une extrémité supérieure pourvue d'une rehausse de chute 702 formée par un disque permettant de limiter le mouvement de translation de la masse 6 le long de la barre de guidage 703, vers le haut. La barre de guidage 703 est reçue dans l'alésage central 65 de la masse 6.

La rehausse de chute 702 est prolongée vers le haut par une poignée 701, permettant à l'utilisateur de maintenir l'ensemble 10 lors de la réalisation d'un essai.

L'extrémité inférieure de la barre de guidage 703 est fixée à un plateau d'impact 707 en forme de disque, par des moyens de fixation 704 formés par un orifice réalisé dans la face supérieure du plateau d'impact 707. La fixation de la barre de guidage 703 sur le plateau d'impact 707 est réalisée par exemple au moyen de vis et/ou de goupilles élastiques.

La face supérieure du plateau d'impact 707 est équipée d'un troisième élément d'amortissement 705 en forme de disque, dont le centre est percé d'un trou pour le passage de la barre de guidage 703. Des éléments de fixation tels que des vis 706 sont utilisés pour fixer le troisième élément d'amortissement 705 sur le plateau d'impact 707.

La face inférieure du plateau d'impact 707 est raccordée à un logement 709 qui délimite une cavité 708 ouverte vers le bas, de forme complémentaire à celle de la partie supérieure de la tête de battage 3. Lorsque le pénétromètre 1 est assemblé avec le dispositif de battage 2, la tête 3 est logée partiellement dans la cavité 708 et le casque 308 est en appui au fond du logement 709. Des moyens de fixation 710 tels que des vis sont insérés dans des orifices du logement 709 orientés radialement, de manière à assurer le maintien en position de la tête de battage 3 dans le logement 709. Les vis 710 sont prévues pour coopérer avec les trous 311 de la tête de battage 3.

Le fonctionnement est le suivant. Lorsque le pénétromètre 1 est utilisé sans le dispositif de battage 2, il s'agit d'un pénétromètre dynamique à énergie variable. Les impacts sont réalisés au moyen du maillet 8, qui est utilisé pour frapper le casque 308 de la tête de battage 3.

Lorsque le pénétromètre 1 est utilisé en combinaison avec le dispositif de battage 2, l'ensemble 10 ainsi formé constitue un pénétromètre dynamique à énergie constante. Les impacts sont réalisés en soulevant la masse 6, en s'aidant des moyens de préhension 63, jusqu'à ce que la masse 6 viennent en butée contre la rehausse de chute 702. Puis, l'opérateur lâche la masse 6, qui coulisse vers le bas le long de la barre de guidage 703 jusqu'à ce que l'élément d'amortissement 61 de la masse 6 impacte l'élément d'amortissement 705 disposé par-dessus le plateau d'impact 707. Le contact entre les éléments d'amortissements 61 et 705 génère un choc qui est atténué, ce qui améliore les mesures.

La hauteur de chute est fixée par la distance entre la rehausse de chute 702 et l'élément d'amortissement 705, qui est constante. Par conséquent, l'énergie d'impact est également constante.

Lors de la réalisation d'un essai à énergie variable ou constante, pour chaque impact donné lors du battage au moyen du maillet 8 ou de la masse 6, l'accéléromètre 305 mesure l'accélération a(t) du pénétromètre 1 en fonction du temps, à une fréquence d'échantillonnage donnée. Ce signal a(t) est transmis au poste d'analyse 9 par l'élément de connexion 309. Le poste d'analyse 9 traite le signal a(t), le stocke et calcule des paramètres relatifs à la compacité du sol S, qui sont affichés sous forme de pénétrogrammes sur un écran du poste d'analyse 9.

Préalablement aux essais de pénétration, on réalise une seule fois une procédure d'étalonnage afin de calibrer l'accéléromètre 305, à l'aide d'un pendule de battage dont la masse est égale à celle du maillet 8 employé. Le pendule de battage est utilisé en orientant le pénétromètre à l'horizontale, et en frappant le casque 308 de la tête de battage 3 au moyen du pendule. La procédure d'étalonnage permet de produire des données d'étalonnage pour le maillet 8 utilisé, indiquant l'énergie de battage en fonction de l'accélération maximum ($a_{pic}(t)$).

Dans le cas du pénétromètre à énergie constante, l'énergie de battage est connue et l'étalonnage est superflu.

Lors d'un essai de pénétration, la force d'impact $F_{pic}(t)$ et l'énergie de battage Eb(t) sont calculées dans une première étape de calcul, à partir du signal a(t) fourni par l'accéléromètre 305 et des données d'étalonnage :

$$F_{pic}(t) = A0.a_{pic}(t) + B0 \qquad \text{(relation 1)}$$

$$Eb(t) = \frac{A1}{B1}.\frac{1}{2}MV^2 + C1 \qquad \text{(relation 2)}$$

avec :

- $a_{pic}(t)$ l'accélération pic mesurée par l'accéléromètre 305 lors de l'impact, c'est-à-dire l'accélération maximale,
- M la masse de la masse 6 ou du maillet 8, enregistrée préalablement dans une mémoire du poste d'analyse 9,
- V la vitesse d'impact de la masse 6 ou du maillet 8 qui est une fonction exponentielle de la force $F_{pic}(t)$.

Les constantes A0, A1, B0 B1 et C1 sont déterminées préalablement lors de la procédure d'étalonnage.

Le signal a(t) est également utilisé par le poste d'analyse 9, dans une deuxième étape de calcul, pour déterminer la distance d'enfoncement s(t) de la pointe 5 dans le sol S, et la vitesse d'enfoncement v(t) de la pointe 5 dans le sol S, pour chaque impact. Ce calcul est réalisé en intégrant une fois ou deux fois le signal a(t), en fonction du temps où selon la fréquence.

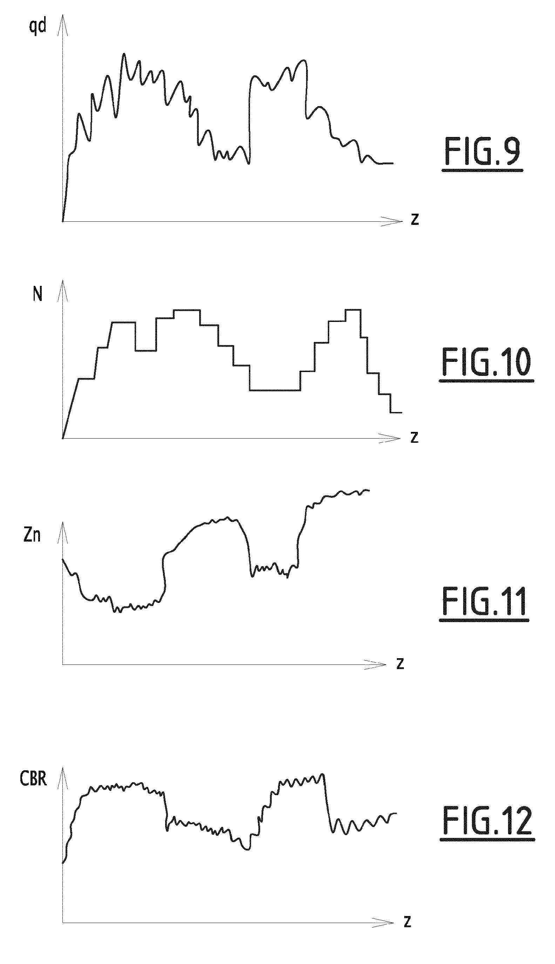

Dans une troisième étape, le poste d'analyse 9 affiche les résultats sous la forme d'un pénétrogramme, tel que celui représenté à la figure 9, montrant l'évolution d'un premier paramètre relatif à la compacité du sol S, à savoir la résistance de pointe qd, en fonction de la profondeur d'enfoncement s de la pointe 5 dans le sol S. De manière classique, une formule mathématique, dite formule des hollandais, permet de calculer la résistance de pointe qd à partir de l'énergie de battage Eb :

$$qd = \frac{Eb}{Ac.e} \cdot \frac{M}{M+P'} \qquad \text{(relation 3)}$$

avec

- qd la résistance en pointe, c'est-à-dire l'effort du sol sur la pointe 5 lors de l'enfoncement d'une pointe conique.
- Eb l'énergie de battage, déterminée précédemment,
- Ac la section projetée de la partie conique 53 de la pointe 5,
- e l'enfoncement de la pointe 5 après chaque impact, qui est une valeur de la distance d'enfoncement s(t),
- M la masse du maillet 8 ou de la masse 6,
- P' la masse battue, c'est à dire la masse du pénétromètre considéré dans son ensemble, à savoir la masse du pénétromètre 1 dans le cas d'un essai à énergie variable, ou la masse de l'ensemble 10 dans le cas d'un essai à énergie constante.

La formule des hollandais est utilisée pour interpréter les enregistrements issus d'un essai, et permet d'évaluer la dureté du sol S, c'est-à-dire sa capacité portante.

La figure 10 montre un deuxième exemple de pénétrogramme, qui indique un deuxième paramètre relatif à la compacité du sol, à savoir le nombre de coups N de maillet 8 ou de masse 6, en fonction de la profondeur d'enfoncement z de la pointe 5 dans le sol S.

La figure 11 montre un troisième exemple de pénétrogramme, qui indique un troisième paramètre relatif à la compacité du sol, à savoir l'enfoncement par coup Zn.

La figure 12 montre un quatrième exemple de pénétrogramme, qui indique un quatrième paramètre relatif à la compacité du sol, à savoir les variations d'un indice CBR, en fonction de la profondeur d'enfoncement z de la pointe 5 dans le sol S. L'indice CBR (Californian Bearing Ratio), où ratio de portance californien, est un paramètre de contrôle de la portance de couche, selon la norme américaine ASTM 6951.

Lors d'un essai, un écran du poste d'analyse 9 affiche le ou les pénétrogrammes obtenus en temps réel, ce qui permet d'obtenir facilement et de manière fiable un paramètre relatif à la compacité du sol.

Le pénétromètre 1, ou plus globalement l'ensemble 10, est équipé uniquement de moyens de mesure de l'accélération, c'est-à-dire qu'il ne comprend pas d'autres moyens de mesure, tels que des moyens de mesure directe de la profondeur d'enfoncement, par exemple un laser placé sur le sol S. Dans l'exemple décrit, il s'agit d'un unique accéléromètre 305, mais en variante le pénétromètre 1 peut incorporer plusieurs accéléromètres et/ou géophones.

Le ou les accéléromètres 305 et le ou les capteurs de mesure de la vitesse sont des moyens de mesure pour déterminer à la fois l'énergie de battage Eb(t) et l'enfoncement s(t) de la pointe 5 dans le sol S. Ces moyens de mesure sont portés en intégralité par la tête de battage 3, aucun élément de ces moyens de mesure n'est disposé au sol ou sur le maillet 8. Les moyens de mesure 305 mesurent, lors d'un essai, uniquement l'accélération a(t) et/ou la vitesse du pénétromètre 1 ou de l'ensemble 10, afin de déterminer l'énergie de battage Eb(t) et l'enfoncement s(t). Aucune mesure additionnelle, notamment une mesure de la force d'impact de la masse 6 ou du maillet 8, ou une mesure directe de l'enfoncement s(t), n'est nécessaire pour calculer l'énergie de battage Eb(t) et l'enfoncement s(t), qui sont calculés uniquement à partir de la mesure de l'accélération et/ou de la vitesse, ainsi que de grandeurs déterminées lors d'un étalonnage préalable, ou par le calcul à partir de l'accélération a(t) et/ou e la vitesse v(t).

Dans le cas de l'utilisation d'un capteur de mesure de la vitesse en remplacement de l'accéléromètre, le signal de mesure de la vitesse est traité par le poste d'analyse 9 pour calculer l'accélération, par une opération mathématique de dérivée temporelle. Le temps est connu par le poste d'analyse 9, notamment au moyen de la fréquence d'échantillonnage du signal.

La géométrie de la tête de battage 3 et de la colonne de battage 7 est spécialement étudiée afin que les éléments d'amortissement 307, 61 et 705 amortissent l'impact du maillet 8 ou de la masse 6, ce qui présente plusieurs avantages, tels que l'augmentation de la durée du choc et la diminution des hautes fréquences sur le signal a(t). Par conséquent, le traitement du signal est amélioré et les bruits entrainés par chaque coup sont réduits, par rapport à des impacts acier sur acier.

Dans le cadre de l'invention, les différentes variantes décrites peuvent être combinées entre elles au moins partiellement.

## Revendications

1. Pénétromètre dynamique (1), comprenant une tête de battage (3), qui est prolongée par une barre (4) terminée par une pointe (5) prévue pour pénétrer dans un sol (S), et des moyens de mesure (305) pour déterminer à la fois l'énergie de battage, appliquée sur la tête de battage, et l'enfoncement de la pointe dans le sol, **caractérisé en ce que** les moyens de mesure (305) sont portés en intégralité par la tête de battage et sont adaptés pour mesurer l'accélération et/ou la vitesse de la tête de battage.

**2.** Pénétromètre (1) selon la revendication 1, **caractérisé en ce que** les moyens de mesure (305) comprennent au moins un accéléromètre, notamment de type piézorésistif ou piézoélectrique.

**3.** Pénétromètre (1) selon la revendication 1, **caractérisé en ce que** les moyens de mesure (305) consistent en un unique accéléromètre ou en un unique géophone.

**4.** Pénétromètre (1) selon l'une des revendications précédentes, **caractérisé en ce que** les moyens de mesure (305) sont logés dans une cavité (314) à l'intérieur de la tête de battage (3).

**5.** Pénétromètre (1) selon l'une des revendications précédentes, **caractérisé en ce qu'**il comprend un élément (309) de connexion des moyens de mesure (305) à un poste d'analyse (9).

**6.** Pénétromètre (1) selon l'une des revendications précédentes, **caractérisé en ce qu'**une enclume (316) de la tête de battage (3), prévue pour être impactée, comprend un premier élément d'amortissement (307) intercalé entre deux parties métalliques (306, 308) de l'enclume (316).

**7.** Pénétromètre (1) selon l'une des revendications précédentes, **caractérisé en ce que** la tête de mesure (3), la barre (4) et la pointe (5) sont solidaires lors d'un essai.

**8.** Pénétromètre (1) selon l'une des revendications précédentes, **caractérisé en ce que** le pénétromètre est dépourvu de moyens de mesure directe de l'enfoncement de la pointe (5) dans le sol.

**9.** Ensemble (10), **caractérisé en ce qu'**il comprend un pénétromètre (1) selon l'une des revendications précédentes, ainsi qu'un dispositif de battage (2) comportant des moyens (708, 709, 710) d'assemblage avec la tête de battage (3), le dispositif de battage (2) comprenant une barre de guidage (703) le long de laquelle coulisse une masse de battage (6) prévue pour impacter un plateau d'impact (707).

**10.** Ensemble (10) selon la revendication 9, **caractérisé en ce que** la masse de battage (6) est équipée d'un deuxième élément d'amortissement (61), prévu pour impacter le plateau d'impact (707).

**11.** Ensemble (10) selon l'une des revendications 9 ou 10, **caractérisé en ce que** le plateau d'impact (707) est équipé d'un troisième élément d'amortissement (705) de l'impact de la masse de battage (6).

**12.** Système, **caractérisé en ce qu'**il comprend un pénétromètre (1) selon l'une des revendications 1 à 8 ou un ensemble (10) selon l'une des revendications 9 à 11, ainsi que des moyens (9) de traitement d'un signal de mesure fourni par les moyens de mesure (305), pour la détermination de l'énergie de battage et de l'enfoncement de la pointe (5) dans le sol (S).

**13.** Méthode de mesure de la compacité d'un sol au moyen d'un pénétromètre (1) selon l'une des revendications 1 à 8, d'un ensemble selon l'une des revendications 9 à 11 ou d'un système selon la revendication 12, **caractérisée en ce qu'**elle comprend:

- une étape d'étalonnage des moyens de mesure (305),
- une étape de mesure, dans laquelle on réalise un essai de pénétration dynamique lors duquel les moyens de mesure (305) mesurent uniquement l'accélération et/ou la vitesse de la tête de battage (3) du pénétromètre (1), des moyens de traitement (9) enregistrant un signal délivré par les moyens de mesure (305),
- une étape de calcul dans laquelle les moyens de traitement (9) déterminent au moins un paramètre relatif à la compacité du sol, à partir du signal délivré par les moyens de mesure (305).

**14.** Méthode selon la revendication 13, caractérisée dans l'étape de calcul, les moyens de traitement (9) calculent l'énergie de battage et l'enfoncement de la pointe (5) dans le sol (S) à partir du signal fourni par les moyens de mesure (305) et en ce que l'énergie de battage et l'enfoncement sont utilisés par les moyens de traitement pour calculer au moins un paramètre relatif à la compacité du sol (S).

**15.** Méthode selon l'une des revendications 13 ou 14, **caractérisée en ce que**, lors de l'essai à l'étape de mesure, ni la force d'impact sur la tête de battage (3), ni l'enfoncement de la pointe (5) dans le sol ne sont mesurées.

**FIG.1**

8

81

82

## FIG.2

5

X1

51

52

53

α

E5b

## FIG.3

5'

X1

51'

52'

53'

α'

E'5b

## FIG.4

**FIG.5**

**FIG.6**

7

X2

701

702

703

706

705

707

704

710

709

708

710

## FIG.7

10

X1,X2

701

702

6

61

2

9

7

705

707

710

303

710

PC

3

315

4

1

S

5

FIG.8

qd

**FIG.9**

Z

N

**FIG.10**

Z

Zn

**FIG.11**

Z

CBR

**FIG.12**

Z

**EP 2 944 725 A1**

Europäisches
Patentamt

European
Patent Office

Office européen
des brevets

# RAPPORT DE RECHERCHE EUROPEENNE

Numéro de la demande

EP 15 16 7264

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2013/124426 A1 (SOL SOLUTION [FR]) 29 août 2013 (2013-08-29) * page 3, lignes 5-7, 16-18, 28-30; revendication 1; figures 1-3 * * page 4, lignes 23-35 * * page 7, lignes 6-7, 22-26; figure 5 * * page 8, lignes 3-12 * * page 7, lignes 12-14; figure 5 * * page 9, ligne 7 - page 10, ligne 8 * ----- | 1-15 | INV. E02D1/02 G01N3/30 G01N3/34 ADD. E21B49/00 G01N11/12 G01N33/24 |
| A | Miguel Angel BENZ-NAVARRETE: "LE GRIZZLY 2® - AUTOMATISATION DES PENETROMETRESDYNAMIQUES ET MESURES D'ENERGIE LORS DU BATTAGE", Journées Nationales de Géotechnique et de Géologie de l'Ingénieur JNGG2012 - Bordeaux, 6 juillet 2012 (2012-07-06), XP002729709, Bordeaux / France Extrait de l'Internet: URL:http://www.geotech-fr.org/sites/defaul t/files/congres/jngg/JNGG-2012-157.pdf [extrait le 2014-09-10] * alinéas [0002] - [0004]; figures 1, 4 * ----- | 1-15 | |
| A | DE 20 2005 018879 U1 (ZORN BERND [DE]) 16 février 2006 (2006-02-16) * alinéa [0017]; figure 1 * ----- | 9-11 | DOMAINES TECHNIQUES RECHERCHES (IPC) E02D E21B G01N |
| A | DE 10 2007 035348 A1 (ZORN BERND [DE]) 5 février 2009 (2009-02-05) * le document en entier * ----- | 9-11 | |

Le présent rapport a été établi pour toutes les revendications

| Lieu de la recherche | Date d'achèvement de la recherche | Examinateur |
|---|---|---|
| La Haye | 14 septembre 2015 | Bamière, François |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 15 16 7264

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-09-2015

| Document brevet cité au rapport de recherche | Date de publication | Membre(s) de la famille de brevet(s) | Date de publication |
|---|---|---|---|
| WO 2013124426 A1 | 29-08-2013 | CL 2014002234 A1<br>EP 2817607 A1<br>FR 2987444 A1<br>US 2015007640 A1<br>WO 2013124426 A1 | 27-03-2015<br>31-12-2014<br>30-08-2013<br>08-01-2015<br>29-08-2013 |
| DE 202005018879 U1 | 16-02-2006 | AUCUN | |
| DE 102007035348 A1 | 05-02-2009 | CN 101354328 A<br>DE 102007035348 A1<br>HK 1127806 A1 | 28-01-2009<br>05-02-2009<br>21-12-2012 |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20070277598 A
- FR 2817344 A
- WO 2013124426 A